# EUROPEAN PATENT SPECIFICATION

(11) **EP 1 935 395 B1**
(45) Date of publication and mention of the grant of the patent: **05.06.2013**
(21) Application number: 07121381.3
(22) Date of filing: 23.11.2007
(51) Int. Cl.: A61Q 11/00, A61K 8/81, A61K 8/02

(54) **Oral composition**
Mundpflegezusammensetzungen
Compositions de protection orale

(30) Priority: 20.12.2006 EP 06126665
(43) Date of publication of application: 25.06.2008
(73) Proprietor: Unilever PLC, London EC4Y 0DY (GB); Unilever N.V., 3013 AL Rotterdam (NL)
(72) Inventor: Brignoli, Cinzia, 26841, Casalpusterlengo (IT); Joiner, Andrew, Bebington, Wirral Merseyside CH63 3JW (GB)
(74) Representative: James, Helen Sarah

(56) References cited:
- WO-A-99/39685
- WO-A-2006/081926
- US-A- 4 456 585
- US-A- 4 518 578
- US-A- 4 627 977
- US-A- 6 030 222
- US-A1- 2005 058 602
- US-A1- 2006 104 922
- US-B1- 6 508 549
- DATABASE CAPLUS [Online] CHEMICAL ABSTRACTS SERVICE, COLUMBUS, OHIO, US; 10 August 2006 (2006-08-10), XP002436273 retrieved from STN Database accession no. 2006:790971
- DATABASE REGISTRY [Online] CHEMICAL ABSTRACTS SERVICE, COLUMBUS, OHIO, US; RN 147-14-8 "Phthalocyanine Blue" XP002436275 retrieved from STN
- DATABASE REGISTRY [Online] CHEMICAL ABSTRACTS SERVICE, COLUMBUS, OHIO, US; RN1328-53-6 16 November 1984 (1984-11-16), "Phthalocyanine green" XP002436274 retrieved from STN
- HANGZHOU UNION PIGMENT COR.: "The life of color in the HUPC" PIGMENT BLUE SERIES UN8617 FAST BLUE B (PIMENT BLUE 15), [Online] 2006, XP002436376 Retrieved from the Internet: URL:http://pigment.global-markings.com/chi na/pigment_blue_15_8617.html> [retrieved on 2007-07-06]

## Description

The present invention relates to oral care compositions comprising a pigment and a deposition aid to enhance the white appearance of teeth.

US 6 030 222 (Tarver) discloses a whitening composition comprising a dye that, when absorbed by a tooth, causes the tooth to reflect a color of light which is whiter than the initial color of light reflected by the tooth.

US 2006/0104922 (Tarver) discloses a tooth whitening system for concealing tooth discoloration. The system comprises a single, spectrally pure, substantially violet dye and a carrier for applying it to the teeth.

The object of the invention is to deliver a whitening benefit to the teeth.

This object is achieved with the claimed compositions.

The amount of pigment in the composition is from 0.01 to 0.3%, preferably from 0.02 to 0.1%, and more preferably from 0.03 to 0.08% by weight. The percentage weights refer to the total amount of active present and exclude any carriers that may be present. The pigment may be uniformly spread throughout the composition or, preferably, it may be dispersed in a second phase such as a stripe or other coextruded second phase. Such preferred "dual phase" compositions have the advantage that the phases may be differently coloured, presenting a more visually attractive product to the consumer.

Preferably, the pigment is violet or blue, more preferably one of those listed in the Colour Index International. These pigments are listed as pigment violet 1 through to pigment violet 56 and pigment blue 1 through 83.

The preferred pigment violets are pigment violet 1, 1:1, 1:2, 2, 3, 5:1, 13, 19, 23, 25, 27, 31, 32, 37, 39, 42, 44 and 50.

The preferred pigment blues are pigment blue 1, 2, 9, 10, 14, 15, 15:1, 15:2, 15:3, 15:4, 15:6 16, 18, 19, 24:1, 25, 56, 60, 61, 62 and 66.

Other suitable pigments are pigment ultramarine blue and ultramarine violet.

The pigment should have a hue angle, h, in the CIELAB system of from 220 to 320 degrees most preferably between 250 and 290 degrees. A detailed description of hue angle may be found on p57 of Colour Chemistry 3rd edition by H. Zollinger published by Wiley-VCH. While the preferred single pigments are blue or violet, the same effect may be achieved through mixing pigments outside of this h range; for example, such a hue angle may also be obtained by mixing a red and green-blue pigment to yield a blue or violet shaded pigment.

Preferably, the pigment is Pigment Blue 15, more preferably Pigment Blue 15:1, 15:2, 15:3, 15:4, 15:5 or 15:6, most preferably 15:1.

Preferably, the pigment is capable of reflecting sufficient light such that the treated tooth is perceivably whiter than its initial colour. Preferably, the pigment is coloured such that its natural colour is within the violet-red to green-blue colour, preferably from violet to blue.

A pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37°C. As a reasonable approximation, the relevant medium may be considered to be water and the relevant temperature to be 25°C.

In the context of this invention, a "soluble" deposition aid is a material that is soluble in water, typically having a solubility of 0.5% or greater, and more typically 5% or greater by weight, at 25°C. Further, such a material remains soluble following drying - i.e. it can be redissolved following drying. Such materials are typically polymers, but are not film-forming polymers. Water solubility is required in order to avoid build up of the deposition aid on the teeth, something that can also be a particular problem with film-forming polymers.

The soluble deposition aid enhances deposition of the pigment onto the teeth and thereby enhances the colour change caused by the pigment. More particularly, a deposition agent in accordance with the invention is able to enhance the deposition of a pigment such as Blue Covarine when incorporated in a composition at a level of 0.02% by weight, particularly when the deposition aid is itself incorporated at a level of 1% by weight.

Without wishing to be bound by theory, it is believed that the insoluble deposition aid works by having affinity for both the pigment and the surface of the teeth, the deposition aid serving as a link between the two.

In a preferred embodiment, the soluble deposition aid is able to enhance the deposition of the pigment onto the teeth by at least 20% and more preferably by at least 100% by effect. In the context of this invention, percentages "by effect" mean that the deposition aid enhances pigment deposition sufficiently for delta b* to be changed by the indicated magnitude - the change being an enhancement of the negative delta b* obtained from use of the pigment in the absence of the deposition aid.

Delta b* is a magnitude of colour change along a yellow-blue axis, negative delta b* corresponding to reduced yellowness.

A simple test for establishing the effect of the deposition aid is as follows.

Polished hydroxyapatite discs are first placed in sterile human saliva for 2 hours to allow a pellicle to form. The discs are then rinsed in water and baseline colour measurements made (using, for example, a Minolta chromameter CR300). The discs are then brushed with (i) a suspension of pigment in water (e.g. Blue Covarine [BC] at 0.02% by weight) or (ii) a suspension of the same pigment at the same concentration as in (i), together with the deposition aid at approximately 50 times said concentration (e.g. BC at 0.02% and deposition aid at 1% by weight). The brushing is best performed using a brushing machine. Following rinsing, the colour of the discs is then re-measured and the change in delta b* is recorded for both treatment (i) and treatment (ii). From a comparison of these data, the effect of the deposition aid is readily seen.

The deposition aid is incorporated into the composition at preferably from 0.01 to 10%, more preferably at from 0.05 to 5%, and most preferably at from 0.1 to 1% by weight.

Deposition aids for use in accordance with the present invention are typically high molecular weight polymers, i.e. polymers having a molecular weight of 200,000 or greater. Deposition aids are selected from Gantrez® type polymers, which are co-polymers of maleic anhydride with methyl vinylether, in which the anhydride moiety may be in a partially or fully hydrolysed or alcoholysed form. Gantrez® polymers themselves are available from ISP Inc. Suitable Gantrez® polymers are:
Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.

Gantrez® type polymers in which the anhydride moiety is fully hydrolysed are preferred. These may be thought of as co-polymers of maleic acid and methyl vinylether. Particularly preferred co-polymers of maleic acid and methyl vinylether have a molecular weight of 1,000,000 or greater and an especially preferred material is Gantrez S-97.

Preferably, the oral care composition comprises water, thickener, surfactant and abrasive. Suitable thickeners include silicas and calcium carbonate. The preferred thickener is silica. Suitable surfactants include the alkali-metal alkyl sulphate surfactants such as the sodium alkyl sulphates, the most preferred being sodium laurylsulphate.

Preferred abrasive materials include silicas, aluminas, calcium carbonates, dicalcium phosphates, calcium pyrophosphates, hydroxyapatites, trimetaphosphates, insoluble hexametaphosphates and so on, including agglomerated particulate abrasive materials, usually in amounts between 3 and 60% by weight of the oral care composition. The most preferred abrasives are calcium carbonate and silica, especially silica.

Preferably, the oral care composition according to the invention comprises a plurality of visually distinct formulations. By this is meant that the composition comprises separate and different formulations which are adjacent one another when extruded from a tube or the like. Typical examples include a composition which comprises a stripe formulation and a base formulation. Another example includes a composition comprising a core formulation and a sheath formulation. The core formulation is preferably located coaxially within the sheath formulation. The term 'coaxially' means substantially central in cross section and is not meant to represent any mathematical accuracy.

Preferably, the oral care composition comprising more than one formulation as previously described comprises at least 90%, preferably at least 95% and most preferably at least 99% by weight of the total pigment in the composition is in one of the formulations.

Where the composition comprises more than one formulation and where one of the formulations comprises the bulk of the pigment as described the remaining formulation(s) is/are preferably translucent or visually clear.

Where the composition comprises more than one formulation, it is preferred that at least 90%, more preferably at least 95% and most preferably at least 99% of any methyl vinyl ether and maleic anhydride copolymer is present in the same formulation as the pigment.

Preferably, the oral care composition comprises a first formulation located co-axially within a second formulation, the first formulation comprising blue pigment and the second formulation comprising a pearlescer, preferably mica.

The oral care compositions according to the invention may comprise further ingredients which are common in the art, such as:
antimicrobial agents, e.g. Triclosan, chlorhexidine, copper, zinc, and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;
humectants such as glycerol, sorbitol, propyleneglycol, xylitol, lactitol etc.;
binders and thickeners such as sodium carboxymethylcellulose, xanthan gum, gum arabic etc. as well as synthetic polymers such as polyacrylates and carboxyvinyl polymers such as Carbopol®;
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Liposomes may also be used to improve delivery or stability of active ingredients.

The oral care compositions may be in any form common in the art, e.g. toothpaste, gel, mousse, aerosol, chewing gum, lozenge, powder, cream, and may also be formulated into systems for use in dual-compartment type dispensers.

Preferred forms are toothpastes and chewing gums. Preferred chewing gum compositions are sugar free and particularly preferred comprise a Gantrez® type polymer.

In the following non-limiting examples, amounts are percentages by weight unless otherwise stated.

### Example 1: Tooth whitening effect of BC compared with dyes (reference)

Teeth (n = 7) were kept in sterile saliva for 1 hour. They were then dipped in a treatment at 0.2% by weight in water for one minute and then immersed in clean water for the times indicated in Table 1. Following these times, delta b* colour measurements were made.

**Table 1**

| **Treatment** | **Time immersed in water (mins.)** | | | | |
|---|---|---|---|---|---|
| | 0 | 10 | 20 | 60 | 120 |
| Patent Blue | -18.2 | -0.5 | -0.3 | -0.4 | -0.2 |
| FD&C | -11.2 | -0.4 | -0.2 | -0.4 | -0.1 |
| Blue Covarine | -7.7 | -2.4 | -2.5 | -2.5 | -2.3 |
| Brilliant Black BN | -12.8 | 0.1 | -0.6 | -0.3 | -0.1 |
| Control | -0.1 | -0.3 | -0.1 | -0.1 | -0.3 |

A negative delta b* indicates a reduction in yellowness. From these data, it is apparent that Blue Covarine pigment remains on the tooth surface for at least 2 hours, whilst the dyes wash off immediately. Values of delta b* less than 1 are not perceivable by eye, so all the dyes were ineffective at causing perceivable colour change.

### Example 2: Toothpaste Composition

Table 2 gives details of a single phase formulation according to an embodiment of the invention.

**Table 2**

| **Component** | | **%** |
|---|---|---|
| Water | | 8.15 |
| Neosorb 70/70 | Aqu. sorbitol (70% ad.) | 66 |
| PEG 32 | | 2 |
| Sodium fluoride | | 0.32 |
| Sodium saccharin | | 0.27 |
| Blue Covarine W6795 | 40% suspension in SLS and glycerol | 0.5 |
| Gantrez S97 | | 0.1 |
| Trisodium phosphate | | 0.11 |
| Tixosil 43 | Thickening silica | 9 |
| Sorbosil AC 77 | Abrasive silica | 8 |
| Sorbosil AC 33 | Abrasive silica | 1.65 |
| SLS | Sodium laurylsulphate | 1.8 |
| DP7043 | Flavour | 1.2 |
| CMC9 | Carboxymethylcellulose | 0.9 |

### Example 3: Dual Phase Toothpaste Composition

Table 3 gives details of a dual phase formulation according to the invention. The first formulation is the sheath and the second formulation is the core of a co-axially, coextruded toothpaste. The core comprises 10% by volume of the total toothpaste.

**Table 3**

| **Component** | **First Formulation** | **Second Formulation** |
|---|---|---|
| Aqu. Sorbitol (70% ad.) | 66 | 66 |
| Sodium fluoride | 2 | 2 |
| Sodium saccharin | 0.32 | 0.32 |
| Blue Covarine W6795 | 0.27 | 0.27 |
| Sodium fluoride | - | 0.5 |
| Gantrez S97 | - | 1 |
| Mica silver MP149 | 0.05 | - |
| Trisodium phosphate | - | 1.1 |
| Thickener silica | 9 | 5.75 |
| Abrasive silica | 8 | 8 |
| Abrasive silica | 1.5 | 3 |
| Cellulose binder | 0.5 | 0.3 |
| Sodium laurylsulphate | 1.8 | 1.8 |
| Flavour | 1.2 | 1.2 |
| Cellulose binder | 0.4 | 0.4 |
| Water | To 100 | To 100 |

### Example 4: Chalk-based toothpaste composition

**Table 4**

| **Component** | **% w/w** |
|---|---|
| Aqu. Sorbitol (70% ad.) | 30.00 |
| Trisodium phosphate | 1.10 |
| Sodium monofluorophosphate | 1.11 |
| Sweetener | 0.27 |
| Preservative | 0.50 |
| Blue Covarine W6795 | 0.20 |
| Gantrez S97 | 0.50 |
| Thickening silica | 3.00 |
| Abrasive silica | 1.00 |
| Perlite | 0.70 |
| FGNC 5AV* | 40.00 |
| Cellulose binder | 0.75 |
| Sodium laurylsulphate | 1.80 |
| Flavour | 1.20 |
| Water | To 100 |

| | |
|---|---|
| * Fine ground natural chalk. Average particle size: 5 microns. | |

### Example 5: Effect of Deposition Aids

Hydroxyapatite discs (10mm diameter) were polished using P1200 paper (Buehler) and placed in sterile human saliva for 2 hours to allow a pellicle to form. Each disc was rinsed in deionised water (Milli Q water, Millipore, USA) and baseline colour measurements were taken using a chromameter CR300 (Minolta). Materials were tested for enhanced Blue Covarine (BC) delivery by preparing 1% w/w solution of the material plus 0.02% w/w BC in deionised water. The hydroxyapatite discs were randomly assigned to either a test group (material + BC) or a control group (0.02% w/w BC only), n = 8 for each group. The discs were placed in a brushing machine and either test or control mixture (10ml) was added. Each disc was brushed for 1 minute using a standard flat trim toothbrush under a brush load of 375g and a speed of 150 strokes/min. The discs were rinsed in deionised water and the colour of the discs was re-measured. Average changes in yellow-blue colour, delta b*, were then calculated.

Table 5 gives details of the materials tested and their effects upon delta b*. It can be seen that each Gantrez® polymer enhanced the deposition of the pigment onto the teeth by at least 100% by effect. It can also be seen that the Polyox 60K (for comparison) substantially enhanced the deposition of the pigment onto the teeth by effect.

**Table 5: Effect of Deposition Aids**

| **Material** | **Δb*** | **Enhancement** | |
|---|---|---|---|
| | | **Units** | **%** |
| Control | -1.4 | - | - |
| Gantrez S-97 | -4.6 | -3.2 | 129 |
| Gantrez S-95 | -4.8 | -3.4 | 143 |
| Gantrez S-96 | -7.9 | -6.5 | 364 |
| Gantrez MS-955 | -10.6 | -9.2 | 557 |
| Polyox 60K (for comparison) | -3.5 | -2.1 | 50 |

In a further study, a range of hydroxypropylmethylcellulose polymers were investigated using the method described above. Table 6 indicates that these materials were less successful, although Walocel HM 4000PA-2910, a high molecular weight hydroxypropylmethylcellulose, did give an enhancement of Blue Covarine deposition of 26% by effect.

**Table 6: Effect of Deposition Aids (for comparison)**

| **Material** | **Viscosity (mPa.s; 20°C; 10 s⁻¹; 2% aqu. soln.** | **Δb*** | **Enhanced** | |
|---|---|---|---|---|
| | | | **Units** | **%** |
| Control | - | -2.5 | - | - |
| Walocel VP-M-52101 | 13.5-19.4¹ | -1.9 | | |
| Walocel VP-M-52113 | 4.5-8.4¹ | -2.2 | | |
| Walocel HM 5PA-2910 | 4.0-6.0² | -0.2 | | |
| Walocel HM 4000PA-2910 | 3200-4800² | -3.4 | -0.9 | 26 |

| | | | | |
|---|---|---|---|---|
| 1. Measured using a rotary viscometer. 2. Measured using an Ubbelohde viscometer. | | | | |

### Example 6: Chewing Gum Composition

Table 7 gives details of a chewing gum composition (sugar free) in accordance with the invention.

**Table 7**

| **Component** | **Amount (% w/w)** |
|---|---|
| Gum Base | 22.00 |
| Polyol Syrup | 55.00 |
| Maltitol Powder | 19.76 |
| Lecithin | 0.20 |
| Glycerin | 2.00 |
| Flavor | 0.80 |
| Blue Covarine W6795 | 0.04 |
| Gantrez S97BF | 0.20 |

## Claims

1. An oral care composition comprising from 0.01 to 0.3% by weight of a pigment having a hue angle, h, in the CIELAB system of from 220 to 320 degrees, **characterized in that** the composition further comprises a water soluble deposition aid for said pigment that is a polymer having a molecular weight of 200,000 or greater, is not a film-forming polymer and is selected from co-polymers of maleic anhydride with methyl vinylether, in which the anhydride moiety may be in a partially or fully hydrolysed or alcoholysed form.

2. An oral care composition according to any of the preceding claims, **characterized in that** the pigment has a hue angle, h, in the CIELAB system of from 250 to 290 degrees.

3. An oral care composition according to any of the preceding claims, comprising water, thickener, surfactant and abrasive.

4. An oral care composition according to claim 3, wherein the thickener is silica.

5. An oral care composition according to any preceding claim, comprising a plurality of visually distinct formulations.

6. An oral care composition according to claim 5, comprising a stripe formulation and a base formulation.

7. An oral care composition according to claim 5, comprising a core formulation and a sheath formulation.

8. An oral care composition according to any of claims 5-7, wherein at least 90% by weight of the total pigment present in the composition is present in one of the formulations.

9. An oral care composition according to claim 7, comprising a first formulation located coaxially within a second formulation, the first formulation comprising blue pigment and the second formulation comprising a pearlescer, preferably mica.

10. An oral care composition according to any preceding claim, wherein the pigment comprises pigment blue 15.

## Patentansprüche

1. Mundpflegezusammensetzung, die 0,01 bis 0,3 Gew.-% eines Pigments umfasst, das einen Farbtonwinkel, h, im CIELAB-System von 220 bis 320 Grad hat, **dadurch gekennzeichnet, dass** die Zusammensetzung außerdem ein wasserlösliches Abscheidungshilfsmittel für das Pigment umfasst, welches ein Polymer mit einem Molekulargewicht von 200 000 oder größer ist, kein filmbildendes Polymer ist und aus Copolymeren von Maleinsäureanhydrid mit Methylvinylether ausgewählt ist, in denen die Anhydrid-Gruppierung in einer partiell oder vollständig hydrolysierten oder alkoholisierten Form sein kann.

2. Mundpflegezusammensetzung gemäß einem der vorangehenden Ansprüche, **dadurch gekennzeichnet, dass** das Pigment einen Farbtonwinkel, h, im CIELAB-System von 250 bis 290 Grad hat.

3. Mundpflegezusammensetzung gemäß einem der vorangehenden Ansprüche, die Wasser, Verdickungsmittel, Tensid und Schleifmittel umfasst.

4. Mundpflegezusammensetzung gemäß Anspruch 3, wobei das Verdickungsmittel Siliciumdioxid ist.

5. Mundpflegezusammensetzung gemäß einem vorangehenden Anspruch, die eine Vielzahl von visuell getrennten Formulierungen umfasst.

6. Mundpflegezusammensetzung gemäß Anspruch 5, die eine Streifenformulierung und eine Grundformulierung umfasst.

7. Mundpflegezusammensetzung gemäß Anspruch 5, die eine Kernformulierung und eine Mantelformulierung umfasst.

8. Mundpflegezusammensetzung gemäß einem der Ansprüche 5 bis 7, wobei wenigstens 90 Gew.-% des gesamten Pigments, das in der Zusammensetzung vorliegt, in einer der Formulierungen vorliegen.

9. Mundpflegezusammensetzung gemäß Anspruch 7, die eine erste Formulierung, die koaxial innerhalb einer zweiten Formulierung lokalisiert ist, umfasst, wobei die erste Formulierung blaues Pigment umfasst und die zweite Formulierung ein Perlmuttglanzmittel, vorzugsweise Glimmer, umfasst.

10. Mundpflegezusammensetzung gemäß einem vorangehenden Anspruch, wobei das Pigment Pigmentblau 15 umfasst.

## Revendications

1. Composition de soin buccal comprenant de 0,01 à 0,3 % en poids d'un pigment ayant un angle de teinte, h, dans le système CIELAB de 220 à 320 degrés, **caractérisée en ce que** la composition comprend en outre un auxiliaire de dépôt soluble dans l'eau pour ledit pigment qui est un polymère ayant un poids moléculaire de 200 000 ou plus, qui n'est pas un polymère filmogène et qui est choisi parmi les copolymères d'anhydride maléique avec du vinyléther méthylique, le groupement anhydride pouvant être sous une forme partiellement ou totalement hydrolysée ou alcoolysée.

2. Composition de soin buccal selon l'une quelconque des revendications précédentes, **caractérisée en ce que** le pigment a un angle de teinte, h, dans le système CIELAB de 250 à 290 degrés.

3. Composition de soin buccal selon l'une quelconque des revendications précédentes, comprenant de l'eau, un épaississant, un tensioactif et un abrasif.

4. Composition de soin buccal selon la revendication 3, dans laquelle l'épaississant est de la silice.

5. Composition de soin buccal selon l'une quelconque des revendications précédentes, comprenant une pluralité de formulations visuellement distinctes.

6. Composition de soin buccal selon la revendication 5, comprenant une formulation à rayures et une formulation de base.

7. Composition de soin buccal selon la revendication 5, comprenant une formulation centrale et une formulation externe.

8. Composition de soin buccal selon l'une quelconque des revendications 5 à 7, dans laquelle au moins 90 % en poids du pigment total présent dans la composition sont présents dans l'une des formulations.

9. Composition de soin buccal selon la revendication 7, comprenant une première formulation située de manière coaxiale à l'intérieur d'une deuxième formulation, la première formulation comprenant un pigment bleu et la deuxième formulation comprenant un composant perlé, de préférence du mica.

10. Composition de soin buccal selon l'une quelconque des revendications précédentes, dans laquelle le pigment comprend du pigment bleu 15.
